(19) Europäisches Patentamt European Patent Office Office européen des brevets

(11) **EP 2 714 109 B1**

(12) **EUROPEAN PATENT SPECIFICATION**

(45) Date of publication and mention of the grant of the patent:
**24.01.2018 Bulletin 2018/04**

(21) Application number: **12727813.3**

(22) Date of filing: **31.05.2012**

(51) Int Cl.:
*A61L 27/22* *(2006.01)*   *A61L 27/52* *(2006.01)*

(86) International application number:
**PCT/EP2012/060277**

(87) International publication number:
**WO 2012/164032 (06.12.2012 Gazette 2012/49)**

(54) **CROSSLINKED GELATIN HYDROGELS**

VERNETZTE GELATIN-HYDROGELE

HYDROGELS DE GÉLATINE RÉTICULÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2011 IT MI20111009**

(43) Date of publication of application:
**09.04.2014 Bulletin 2014/15**

(73) Proprietor: **Politecnico di Milano**
**20133 Milano (IT)**

(72) Inventors:
  • **TANZI, Maria Cristina**
    **20133 Milano (IT)**
  • **FARE', Silvia**
    **20133 Milano (IT)**
  • **GERGES, Irini**
    **20133 Milano (IT)**

(74) Representative: **Banfi, Paolo**
    **Bianchetti Bracco Minoja S.r.l.**
    **Via Plinio, 63**
    **20129 Milano (IT)**

(56) References cited:
• **MOHAMMAD SADEGHI ET AL: "Crosslinked Graft Copolymer of Methacrylic Acid and Gelatin as a Novel Hydrogel with pH-Responsiveness Properties", MATERIALS, vol. 4, no. 3, 2 March 2011 (2011-03-02), pages 543-552, XP055014017, DOI: 10.3390/ma4030543**
• **SANDRA VAN VLIERBERGHE ET AL: "Porous Gelatin Hydrogels: 1. Cryogenic Formation and Structure Analysis", BIOMACROMOLECULES, vol. 8, no. 2, 1 February 2007 (2007-02-01), pages 331-337, XP055014023, ISSN: 1525-7797, DOI: 10.1021/bm060684o cited in the application**
• **MANJU KUMARI ET AL: "Adsorption capacity, kinetics, and mechanism of copper(II) uptake on gelatin-based hydrogels", JOURNAL OF APPLIED POLYMER SCIENCE, vol. 119, no. 1, 5 January 2011 (2011-01-05), pages 363-370, XP055013999, ISSN: 0021-8995, DOI: 10.1002/app.32632**

## Description

The present invention is situated in the field of regenerative medicine, and in particular relates to the regeneration of soft tissues. More specifically, the invention relates to hydrogel matrices and their biomedical applications as systems for the controlled release of medicaments or for cell culture, delivery and implantation.

## Background to the invention

Hydrogels are polymer materials suitable for use as substrates for the regeneration of soft tissues due to their low mechanical characteristics and high hydrophilicity. They are three-dimensional macromolecular polymer networks, able to absorb a large quantity of aqueous solutions or biological fluids, without being subject to solubilisation.

Among the various types of hydrogels, those containing gelatin have proved to be very promising materials in the biomedical sector. Gelatin is a product of natural origin, derived from collagen, which aids cell adhesion but does not present immunogenicity under physiological conditions, is highly biocompatible, completely resorbable *in vivo*, inexpensive and readily available. The main drawback associated with gelatin is its low mechanical properties, which limit its possible uses as a biomaterial in the non-crosslinked state.

In fact, although gelatin can spontaneously form a gel in aqueous solution at low temperature (< 5-10°C), it presents thermoreversible behaviour, ie. it liquefies again at temperatures exceeding 30°C. Consequently, in order for it to be used as a biomaterial in the biomedical field, it is necessary to increase its stability and mechanical properties under physiological conditions. The formation of inter- and intra-molecular covalent bonds (crosslinking) is therefore important to improve some properties, such as density and solubility, to facilitate control of its degradation and the possible release of substances *in vivo*, and in general to improve its thermal, mechanical and chemical stability in a biological environment.

The scientific literature describes a number of methodologies for crosslinking gelatin, using two different crosslinking methods, physical and chemical.

Physical crosslinking, by UV irradiation or heat treatment, presents the advantage of not introducing external chemical agents which are potentially toxic for the body, but is not very effective, and makes it difficult to control the crosslinking stability and density in the final matrix.

Chemical crosslinking includes the use of chemical agents, which are divided into two classes: non-zero length (such as aldehydes, epoxides and isocyanates) and zero length (such as acyl azides and carbodiimides), and involves the formation of covalent bonds through a condensation reaction, as described by Kuijpers A.J., et al., Journal of Biomaterials Science, Polymer Edition 11 (3) 225-243, 2000.

## State of the art

The literature describes products based on crosslinked gelatin and the use of methylenebisacrylamide (MBA) as crosslinker. See for example A.J. Kuijpers et al, J Biomater Sci Polymer Edn, 2000, 11(3):225-24; P. Dubruel et al, Biomacromolecules 2007, 8:338-344; S. Van Vlierberghe et al, Biomacromolecules 2007, 8:331-337.

WO 2010/060104 A2 discloses semisolid implantable matrices comprising a hydrogel, which are useful for carrying liposomes containing bioactive materials to sites or tissues of interest. The matrix can consist of various biodegradable materials, among which gelatin is mentioned, and can also contain a crosslinker able to form a semisolid matrix with the hydrogel. The various crosslinking agents mentioned include N,N'-methylenebisacrylamide (MBA). However, the document does not describe a gelatin hydrogel crosslinked with N,N'-methylenebisacrylamide.

WO 2008/121422 A2 discloses semisolid implantable matrices containing monocyte cells and comprising a material with the characteristics of a hydrogel. Gelatin is mentioned among the various possible hydrogels. The matrix can also contain a crosslinker, selected from a list that includes MBA. Once again, the document does not disclose a gelatin hydrogel crosslinked with N,N'-methylenebisacrylamide.

Sadeghi M et al, Materials vol. 4, no. 3 (p. 543-552) and Kumari M et al, Journal of Applied Polymer Science, vol. 119 no. 1 (p. 363-370) report the preparation of gelatin-based hydrogel crosslinked with MBA. In both cases, the cross-linking reaction between gelatine and N,N'-methylenebisacrylamide involves a radical mechanism and generates carbon-carbon non hydrolysable bonds. In contrast, the Michael-type reaction according to the present invention proceeds via a nucleophilic addition of free amino groups present in the gelatin and the $\alpha,\beta$ unsaturated carbonyl group in the bisacrylamide.

Van Vlierberghe S et al, Biomacromolecules vol. 8 no. 2 (2007), p. 331-337, disclose the reaction between gelatine B and methacrylic anhydride, wherein the cross-linking takes place on the gelatine carbon-chain without involving lisine moieties.

## Description of the invention

The invention provides gelatin hydrogels crosslinked with bisacrylamides obtained by functionalisation of primary or secondary amines, which possess advantageous mechanical and stability properties in a physiological environment and are particularly advantageous for use in cell culture, growth and proliferation, for the controlled release of bioactive molecules, and to promote tissue regeneration.

Specifically, subject of the present invention is gelatin hydrogels crosslinked with bisacrylamides of primary or secondary amines, obtainable by a Michael-type addition reaction process via the nucleophilic addition of free amino groups in gelatin (lateral lysine groups and the few terminal amine groups) and the $\alpha,\beta$ unsaturated carbonyl group in the selected bisacrylamide, said process comprising the following steps:

(a) reaction of gelatin with bisacrylamide in a water medium;
(b) purification of the product obtained at step (a);
(c) optional drying or lyophilization.

The bisacrylamides of primary or secondary amines are preferably selected from N,N'-methylenebisacrylamide (MBA), N,N'-ethylenebisacrylamide, bis-acryloylpiperazine and bis-acryloylcystamine (CH2=CH- (C=O)-NH-S-S-NH-(C=O)-CH=CH2) (crosslinking agents). Water-soluble bisacrylamides obtainable by functionalisation of homobifunctional oligomers such as polyoxyalkylenediamine using the Schotten-Bauman reaction (N,N'-polyoxyalkylene bisacrylamide) can also be used. The crosslinking agent N,N'-methylenebisacrylamide (MBA) is most preferred.

The gelatin used to prepare the hydrogels according to the invention can be of different origin. Type A gelatin is preferably used.

The reaction is conducted in the presence of a tertiary amine compound with low molecular weight or an inorganic base to obtain a pH suitable for deprotonation of the primary amine groups of the gelatin, and their activation in the presence of the crosslinking agent.

The reaction is conducted in aqueous solution, using gelatin concentrations ranging between 5 and 40% w/v, with a molar ratio of between 0.5 and 2.5 between the amine-equivalent groups present in the gelatin (mM ($NH_2$) lysine) and the acrylamide-equivalent groups present in the crosslinking agent. The operations are performed at a temperature between 30 and 70°C, preferably between 40 and 50°C, in the presence of triethylamine (0.1-0.3 ml/g of gelatin), or a similar tertiary amine compound with a low molecular weight such as triethanolamine, triethylenediamine or N,N,N', N'-tetraethylenediamine (TEMED), or an inorganic base, such as NaOH or LiOH, preferably LiOH monohydrate. The crosslinking agent is added last to the reaction mixture, under stirring. The reaction mixture is then transferred to a stove thermostated at a temperature between 30 and 70°C, preferably between 40 and 50°C, and left to react for between 4 and 24 hours, preferably 12 hours.

The hydrogels thus obtained are subjected to a washing process specifically designed to eliminate any excess of the crosslinking agent and other unreacted constituents, consisting of successive washes with pure ethanol, 0.1M HCl solution, and finally with distilled water.

The hydrogels according to the invention can be stored in an aqueous environment until use, or more preferably under anhydrous conditions, after stove-drying to a constant weight or freeze-drying.

A further subject of the invention is therefore a composition containing, in an aqueous medium or in dried form, a gelatin hydrogel crosslinked with a bisacrylamide as defined above.

The hydrogels according to the invention are obtained under mild conditions, thus avoiding high temperatures, organic solvents, or the use of complex chemical reactions or compounds which can release potentially toxic residues. The Michael addition reaction used to prepare the hydrogels according to the invention is particularly advantageous because it can be conducted at ambient temperature or just above, in a short time and in aqueous solution.

In addition, the hydrogels of the invention can be hydrolysed in physiological conditions and with a degradation kinetics suitable for tissue regeneration. The degradation of the amide groups occurs by hydrolysis in physiologic conditions and it is induced by M+ mesomeric electron delocalization from the secondary amino groups to the carbonyl groups in beta position.

None of the gelatin crosslinking mechanisms previously reported in the literature take place by the Michael addition procedure described in the present invention.

The hydrogels according to the invention are insoluble in aqueous solvents and are able to increase their volume and weight by up to 3 orders of magnitude. They were characterised by stability tests in aqueous solutions and in a physiological environment at 37°C (swelling and weight loss), and by dynamic-mechanical characterisation.

The results of the dynamic-mechanical tests demonstrate that hydrogels based on the gelatin according to the invention have better properties than the comparison materials not containing gelatin.

The synthesis methodology used to prepare the hydrogels according to the invention produces bio- and haemocompatible matrices with much higher stability in a physiological environment (over 35 days) than similar substances already present on the market or undergoing trials.

The hydrogels according to the invention have advantageous characteristics in terms of cytotoxicity and cytocompatibility *in vitro* which makes them suitable for use as cell culture, growth and proliferation media. In particular, the hydrogels according to the invention guarantee a micro-environment suitable for cell adhesion and subsequent maintenance of the ideal conditions for the cell culture, including adult stem cells isolated from adipose tissue.

A further subject of the invention is therefore the use of a crosslinked gelatin hydrogel as defined herein, or of a composition containing it, for cell culture, differentiation or proliferation.

The hydrogels and compositions according to the invention can be used as substrates to promote *in vivo* or *in vitro* regeneration of soft tissues, such as adipose tissue, vascular tissue and skin. They are also useful in the field of controlled release of medicaments, in particular polypeptides and active biomolecules, such as growth factors. They are also suitable for use as cell carriers.

Further subjects of the invention are therefore a crosslinked gelatin hydrogel as defined herein, containing a biologically active substance or cells, and the use of a crosslinked gelatin hydrogel as defined herein, or of a composition containing it, for in vitro regeneration of soft tissues, controlled release of medicaments, polypeptides and active biomolecules, and as a cell carrier.

A further aspect of the invention relates to implantation or grafting of tissues or cells, in particular adult stem cells isolated from adipose tissue, wherein said cells are contained in a hydrogel as defined herein.

The invention will now be illustrated in greater detail by the following examples.

## Example 1 - Experimental synthesis procedure and examples of products

All the reagents used were obtained from Sigma Aldrich: MBA, code 146072, type A porcine gelatin, code G2500-100G, hexamethylenediamine, code HI 1696, and triethylamine, code T0886. The reaction solvent is distilled water (Idrochimica Srl, Milan).

A material synthesised by substituting gelatin with an aliphatic diamine, hexamethylenediamine (HEX), was used as comparison material; said material is indicated by the code MBA/HEX.

Different hydrogels were synthesised by varying the quantity of the reagents. For example, Table 1 shows three different synthesis methods. The comparison product, not containing gelatin, is indicated by the code MBA/HEX.

The experimental procedure was as follows:

A 15% solution (15MBA47, 15MBA23) or a 25% solution (25MBA47) w/v of gelatin A in distilled water was poured into a Petri dish (ø=5 cm), selected as reaction vessel, and heated to 50°C on a thermostated plate; 0.1 ml of triethylamine was then added under stirring. MBA in the quantities reported in Table 1 was added after 30 minutes, again under stirring at 50°C. After a few minutes' stirring, the synthesis mixture was transferred to a stove thermostated at 50°C and left overnight. At the end, the product of reaction underwent three successive washes: immersion for 12 hours in pure ethanol, replaced with fresh ethanol every two hours; immersion in 0.1M HCl for one hour; immersion in distilled water for 12 hours, with replacement every two hours, followed by immersion in distilled water overnight.

When the washing process was finished, a manual punch of a diameter suitable to obtain specimens of appropriate dimensions for the subsequent characterisation tests was used.

*Table 1: Codes and quantities of reagents used to synthesise the different hydrogel samples*

| hydrogel | GELATIN type A | | MBA | | HEX | | TEA |
|---|---|---|---|---|---|---|---|
| | mg | mM (NH$_2$ lysine) | mg | mM | mg | mM | ml |
| **MBA/HEX** | - | - | 1000 | 6,490 | 367,52 | 3,24 | 0,1 |
| **15MBA47** | 1000 | 0,31 | 47,8 | 0,310 | - | - | 0,1 |
| **25MBA47** | 1000 | 0,31 | 47,8 | 0,310 | - | - | 0,1 |
| **15MBA23** | 1000 | 0,31 | 23,9 | 0,155 | - | - | 0,1 |

The estimate of the amine groups present in the gelatin was determined experimentally by titration, according to the method described by Cayot P. et al, Analytical Biochemistry 249, 184-200, 1997.

## Example 2 - Swelling and weight loss tests

The tests were conducted on specimens with initial dimensions ø=12 mm and h=5 mm, stove-dried at 30°C to constant weight. Three specimens were used for each type of hydrogel synthesised. The dry weight of each one was measured before beginning the test. Each specimen was immersed in distilled water and placed in a stove thermostated at 37°C throughout the duration of the test. The time points considered were between 5 and 840 hours (35 days). At each time point the specimens were weighed, and the weight values in the dry and swollen states were used to calculate the weight variation according to the following formula:

$$\Delta Weight\ \% = \frac{W_{timepoint} - W_{dry}}{W_{dry}} \times 100$$

where $W_{timepoint}$ is the swollen weight of the sample at the timepoint considered and $W_{dry}$ is the dry weight.

The weight variation of each type of hydrogel analysed was expressed as the mean and standard deviation of the values obtained for the three specimens of the same type.

The swelling process was conducted in distilled water and in buffered saline solution (Phosphate Buffered Saline, PBS, pH=7.4, Sigma, P4417), to establish the stability at physiological pH.

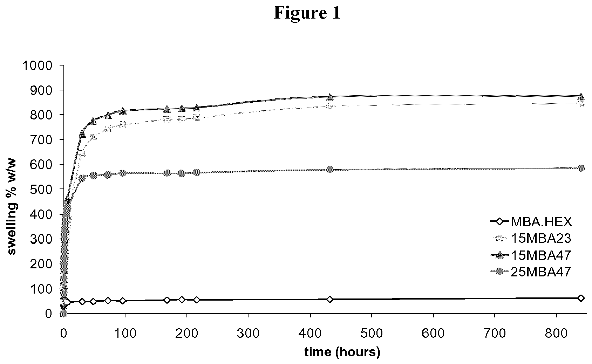

Figure 1 shows the weight variation values measured in PBS. All the hydrogels based on gelatin/MBA proved stable in a physiological environment *in vitro* for up to 35 days, presenting rapid swelling kinetics in the first few hours of the test, reaching a plateau between 50 and 100 hours after immersion, with no weight reductions for up to 35 days. The swelling value at plateau was the greatest (approx. 800%) for the hydrogels synthesised under the highest dilution conditions, whereas in the case of synthesis at higher concentrations, the maximum swelling value remained around 550%. No differences were observed between hydrogels synthesised with different quantities of MBA, the concentration of the reaction mixture being equal. Conversely, the comparison material (MBA-HEX) proved stable at the test times considered, but the swelling values were very low (not exceeding 50%).

## Example 3 - Spectroscopic characterisation

The spectroscopic characterisation of the gelatin/MBA hydrogels was performed by Fourier transform infrared spectroscopy (FTIR), using the Nicolet 6700 spectrometer (Thermo Electron Corporation). The analysis was conducted with the Single Bounce-ATR (Attenuated Total Reflectance) accessory, which allows the study of small surfaces. The samples analysed by FTIR were previously stove-dried under vacuum at 37°C for 72 hours, in such a way as to eliminate from the spectrum the contribution made by the water present in the specimens. The FTIR analyses were conducted with the following experimental parameters: spectral window = 4000-600 $cm^{-1}$, number of scans = 64, crystal = ZnSe.

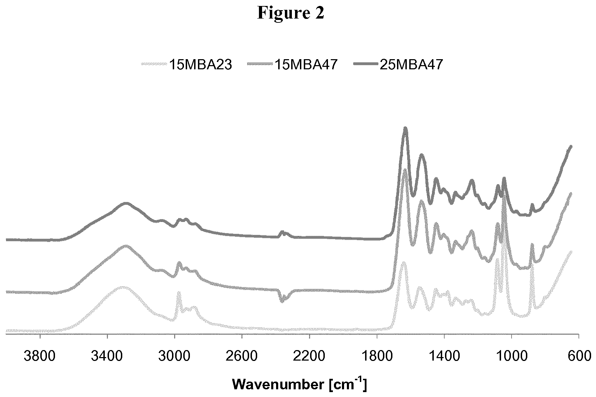

IR spectroscopy allowed the presence of the bands relating to the functional groups characteristic of formation of amide-amine bonds to be verified (stretching of N-C bond of the secondary amines at 1220- 1047 $cm^{-1}$ and tertiary amines at 1111-1087 $cm^{-1}$; stretching of the C=O of the amide bond at 1548 $cm^{-1}$; angle deformation of the N-H amide bond at 1642 $cm^{-1}$). The IR spectra of the hydrogels obtained in example 1 are shown in Figure 2.

## Example 4 - Mechanical properties

The mechanical properties of the gelatin/MBA hydrogels were studied with a dynamic mechanical analyser (DMA mod. 2980, TA Instruments), using mechanical compression tests in frequency sweep and stress relaxation tests with subsequent strain recovery. All the mechanical tests were conducted on cylindrical specimens (ø = 5 mm, h = 3 mm) previously brought to a swelling plateau in distilled water. Three specimens were characterised for each formulation of the gelatin and MBA hydrogels and for the MBA/HEX formulation used as comparator.

*Mechanical compression tests*

Compression tests were performed in frequency sweep (f = 0.5 - 10 Hz, T = 37°C, n = 10 cycles) at a constant temperature (37°C) to obtain the complex modulus trend on variation of frequency. In particular, information can be obtained about the real component of the complex modulus, known as the conservative modulus (E'), which represents the energy accumulated in the material and consequently correlated with its elastic behaviour, and the imaginary component, called the dissipative modulus (E"), which represents the dissipated energy linked to the viscous component of the material. The E"/E' ratio, called *tan delta,* allows the contribution made by the two components to be evaluated on variation of the test parameters.

In the dynamic mechanical compression tests, the hydrogels according to the invention presented conservative and dissipative modulus values that varied according to their chemical composition. As shown in Table 2, the conservative modulus (E') was between 4 and 21 kPa.

EP 2 714 109 B1

*Table 2 - Conservative modulus values (E') for gelatin-based hydrogels according to the invention and for the control material (MBA-HEX)*

| | f [Hz] | cycle #1 | cycle #5 | cycle #10 | | f [Hz] | cycle #1 | cycle #5 | cycle #10 |
|---|---|---|---|---|---|---|---|---|---|
| **MBA-HEX** | 0.5 | 9.97±7.41 | 9.33±7.50 | 9.10±7.42 | **15MBA47** | 0.5 | 13.68±3.49 | 13.32±3.53 | 13.09±3.52 |
| | 1 | 10.17±7.50 | 9.47±7.63 | 9.23±7.56 | | 1 | 14.24±3.60 | 13.77±3.63 | 13.56±3.58 |
| | 2 | 10.40±7.64 | 9.67±7.75 | 9.44±7.67 | | 2 | 14.76±3.64 | 14.36±3.70 | 14.02±3.76 |
| | 3 | 10.80±7.75 | 9.96±7.88 | 9.73±7.83 | | 3 | 15.12±3.82 | 14.72±3.83 | 14.42±3.91 |
| | 4 | 11.19±8.02 | 10.31±8.17 | 10.08±8.05 | | 4 | 15.46±3.93 | 15.01±3.98 | 14.71±3.99 |
| | 5 | 11.59±8.27 | 10.72±8.43 | 10.38±8.35 | | 5 | 15.78±4.17 | 15.32±4.15 | 15.01±4.19 |
| | 6 | 12.09±8.50 | 11.09±8.64 | 10.80±8.53 | | 6 | 16.17±4.10 | 15.65±4.18 | 15.23±4.24 |
| | 7 | 12.56±8.82 | 11.43±9.02 | 10.98±9.05 | | 7 | 16.23±4.27 | 15.57±4.48 | 15.47±4.38 |
| | 8 | 13.03±8.99 | 11.73±9.38 | 11.60±9.20 | | 8 | 16.44±4.33 | 15.93±4.41 | 15.70±4.41 |
| | 9 | 12.94±8.51 | 12.20±9.41 | 12.01±9.54 | | 9 | 16.84±4.45 | 16.21±4.64 | 15.86±4.50 |
| | 10 | 13.70±9.44 | 12.45±9.48 | 12.33±9.34 | | 10 | 16.87±4.23 | 16.14±4.23 | 15.96±4.62 |
| **15MBA23** | 0.5 | 17.91±0.66 | 17.91±0.33 | 17.91±0.12 | **25MBA47** | 0.5 | 4.65±0.09 | 4.33±0.08 | 4.00±0.20 |
| | 1 | 18.18±0.83 | 18.11±0.54 | 18.11±0.18 | | 1 | 4.83±0.13 | 4.54±0.04 | 4.18±0.15 |
| | 2 | 18.45±0.76 | 18.45±0.51 | 18.42±0.27 | | 2 | 4.99±0.17 | 4.67±0.03 | 4.33±0.14 |
| | 3 | 18.75±1.06 | 18.72±0.79 | 18.72±0.50 | | 3 | 5.13±0.28 | 4.78±0.05 | 4.45±0.08 |
| | 4 | 19.09±1.14 | 19.04±0.79 | 19.10±0.58 | | 4 | 5.11±0.29 | 4.90±0.10 | 4.49±0.12 |
| | 5 | 19.34±1.23 | 19.28±0.94 | 19.34±0.71 | | 5 | 5.25±0.37 | 4.99±0.13 | 4.65±0.13 |
| | 6 | 19.44±1.07 | 19.40±0.98 | 19.49±0.58 | | 6 | 5.45±0.21 | 5.04±0.22 | 4.72±0.13 |
| | 7 | 19.54±1.26 | 19.48±0.92 | 19.62±0.68 | | 7 | 5.50±0.28 | 5.15±0.15 | 4.76±0.06 |
| | 8 | 19.84±1.25 | 19.70±1.00 | 19.87±0.48 | | 8 | 5.37±0.60 | 5.31±0.30 | 4.85±0.04 |
| | 9 | 20.09±1.34 | 19.69±1.16 | 20.21±0.59 | | 9 | 5.72±0.05 | 5.79±0.60 | 5.26±0.35 |
| | 10 | 20.08±1.41 | 20.53±0.40 | 20.45±0.64 | | 10 | 5.75±0.67 | 5.71±0.23 | 5.10±0.42 |

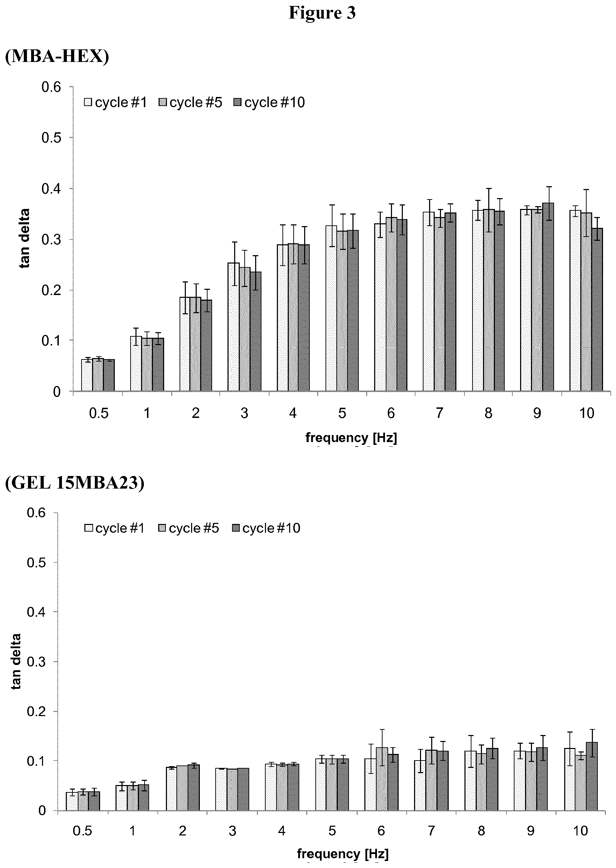

For all the gelatin-based hydrogels according to the invention, a prevalence of the elastic component was observed as the stress frequency and the number of test cycles increased, whereas for the control material, the viscous component prevailed as frequency increased. This behaviour is clearly shown in the graphs in Figure 3, which show the *tan delta* trend in three different test cycles, on variation of the stress frequency for the comparison material (MBA-HEX) and for hydrogel 15MBA23.

*Stress relaxation and deformation recovery tests*

Stress relaxation tests were conducted followed by recovery of deformation in compression at 37°C. Constant deformation (5%) was applied to the samples, and the trend over time of the stress required to maintain the deformation value imposed was studied; when the deformation was removed, the ability to recover the original size was studied. Five stress relaxation cycles were performed, each followed by deformation recovery, to obtain the stress and deformation trends over time.

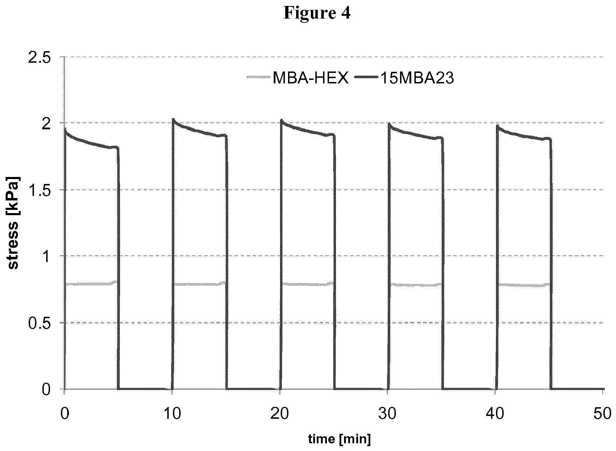

Slight stress relaxation was observed for all the gelatin-based hydrogels according to the invention, which remained constant for the 5 test cycles; moreover, although residual deformation remained after the first cycle, it was completely eliminated in the subsequent cycles. This behaviour indicates that the elastic component prevails under cyclical or repeated stress conditions. Conversely, the comparison material did not show a viscous component, but appeared much weaker. For example, Figure 4 shows the trend of stress relaxation in compression for the comparison material (MBA-HEX) and for hydrogel 15MBA23.

The dynamic mechanical test results indicate that the hydrogels based on gelatin present the best overall properties compared with the comparison material not containing gelatin. Synthesis conditions such that gelatin-based hydrogels present a behaviour wherein the elastic component prevails over the viscous component can also be selected.

### Example 5 - Tests of indirect cytocompatibility with cell line L929

*In vitro* cytotoxicity tests were performed to verify that no products with low molecular weight and/or reaction residues were released. These products, if present, can be released into the cell growth environment and influence cell activity, even causing cell death.

The viability of murine fibroblasts in the L929 line cultured for 24 hours in contact with culture medium wherein the hydrogels were immersed for different times (eluted at 4-168h) was evaluated by MTT colorimetric assay (M5655, Sigma).

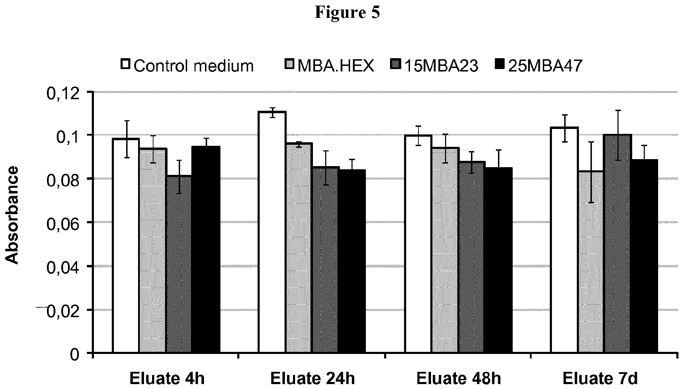

The hydrogels proved not to be cytotoxic; in fact, cells cultured in the presence of eluates obtained for up to 7 days' immersion presented viability levels not significantly different from those obtained for cells cultured in clean culture medium (control), as shown in Figure 5.

### Example 6 - Tests of interaction with adipose tissue stem cells (ASC)

Tests of interaction *in vitro* with stem cells isolated from adipose tissue obtained by liposuction from patients who underwent mastectomy were performed. The ASC cells were isolated according to the protocol described by Rigotti et al, Clinical treatment of radiotherapy tissue damage by lipoaspirate transplant: a healing process mediated by adipose derived adult stem cells, Plast Reconstr Surg. 2007; 119(5):1409-1422, by enzymatic digestion of fatty tissue and centrifugation cycles. The cells were cultured on the hydrogels, differentiation being induced by Adipogenic Induction Medium (AIM) in Dulbecco's Modified Eagle's Medium DMEM (Ham's F12 + 1% Human Serum Albumin) and the cells subsequently being cultured in Adipogenic Differentiation Medium (ADM) in DMEM (Ham's F12 + 1% Human Serum Albumin). Differentiation into adipocytes was studied by evaluating the presence of the lipid drops produced and detected by Oil Red O staining (Sigma-Aldrich).

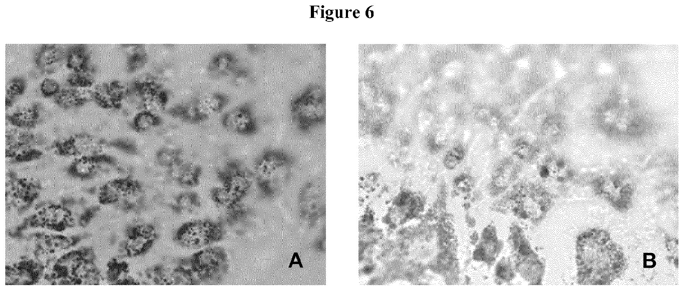

7 days after seeding, Oil Red O staining confirmed the differentiation with adipocyte formation, as shown in Figure 6.

In view of the findings reported in the literature (Tanzi MC et al, Adipose tissue engineering: state of the art, recent advances and innovative approaches. Expert Rev Med Devices. 2009; 6(5):533-51), this result can be considered very good.

### Example 7 - Tests of interaction with human umbilical vein endothelial cells (HUVEC)

The purpose of these cell interaction tests was to establish the suitability of the hydrogels according to the invention to regenerate blood vessels and, in general, for applications in contact with the cardiovascular system.

Two different hydrogels were prepared: gelatin/MBA 25MBA47 and 15MBA23.

After a purification procedure conducted under laminar-flow hood, the samples (n = 3) were dried to constant weight and then swollen in an aqueous solution containing 0.02% w/v of a recombinant elastin (HELP) with a similar sequence to that of human elastin (Bandiera A, et al., Biotechnol Appl Biochem. 2005; 42(Pt 3): 247-56), thus obtaining interpenetrating gelatin/HELP lattices.

These hydrogels were then seeded with HUVEC cells (human umbilical vein endothelial cells) cultured in Endothelial Cell Growth Medium $MV_2$ (basal medium; Promocell, Heidelberg, Germany). 48 hours after seeding, the cultures were fixed with 4% v/v formalin and stained with toluidine blue.

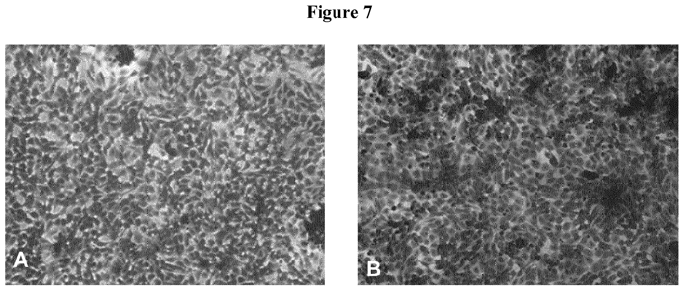

Once again the results were very good: for all samples analysed, the formation of a homogenous confluent single layer was observed (Figure 7), indicating the suitability of these matrices for applications involving the reconstruction and treatment of vascular tissue.

## Description of Figures

**Figure 1** - Degree of swelling in a physiological environment at 37°C of gelatin/methylenebisacrylamide hydrogels obtained with various formulations, and of the comparison material MBA-HEX.

**Figure 2** - FTIR spectra of three gelatin/MBA hydrogels obtained with different formulations.

**Figure 3** - trend of the *tan delta* parameter in three different test cycles, on variation of stress frequency, for the comparison material (MBA-HEX) and hydrogel 15MBA23.

**Figure 4** - Curves representing the trend of stress relaxation in compression (5% constant deformation) for the comparison material (MBA-HEX) and hydrogel 15MBA23.

**Figure 5** - Results of MTT viability assay performed on L929 cells in contact with eluates obtained at different incubation times with the various types of hydrogels. The data relating to the culture medium only are shown in white.

**Figure 6** - Oil Red O staining of ASCs differentiated into adipocytes on a) 15MBA23, and b) 15MBA47. Enlargement: 500X.

**Figure 7** - HUVEC cultures on gel (A) 25MBA47 and (B) 15MBA23 conditioned with 0.02% w/v HELP 24 hours after seeding. Stain: toluidine blue. Enlargement: 100X.

## Claims

1. Hydrogel of gelatin cross-linked with bisacrylamides of primary or secondary amines which are selected from N,N'-methylene-bisacrylamide (MBA), N,N'-ethylene-bisacrylamide, bisacryloylpiperazine, bisacryloylcystamine and N,N'-polyoxyalkylen-bisacrylamide, wherein said hydrogel is obtainable by a Michael-type addition reaction process via the nucleophilic addition of free amino groups in gelatin and the $\alpha,\beta$ unsaturated carbonyl group in the selected bisacrylamide, said process comprising the following steps:

   (a) reaction of gelatin with bisacrylamide in a water medium;
   (b) purification of the product obtained in step (a);
   (c) optional drying or lyophilisation.

2. Hydrogel according to claim 1, wherein the reaction of step (a) comprises:

   (a') preparing an aqueous solution of gelatin at a temperature from 30 to 70°C in the presence of a tertiary amine compound or an inorganic base, wherein the gelatin concentration is between 5 and 40% wt/vol;
   (b') adding bisacrylamide to the solution obtained in (a') in such an amount that the molar ratio between the amino groups present in the gelatin (mM ($NH_2$) lysine) and the acrylamide groups present in the bisacrylamide ranges from 0.5 to 2.5;
   (c') heating the solution obtained in (b') at a temperature of between 30 and 70°C for a time period of 4 to 24 hrs.

3. Hydrogel according to claim 1-2, wherein the purification of step (b) comprises consecutive washings with ethanol followed by HCl 0.1 M and by water.

4. Hydrogel according to claim 1-3, wherein said bisacrylamide is N,N'-methylene-bisacrylamide (MBA);

5. Hydrogel according to claims 1-4 wherein said tertiary amine is triethylamine in amounts ranging from 0.1 to 0.3 ml per gram of gelatin.

**6.** Hydrogel according to any preceding claim, containing a biologically-active substance or cells.

**7.** Composition containing a hydrogel according to claims 1-6 in an aqueous medium or in dry form.

**8.** Hydrogel according to claims 1-6 or a composition according to claim 7 for use in the controlled release of drugs, polypeptides or active biomolecules.

**9.** Hydrogel or composition according to claim 8, wherein said active biomolecules are growth factors.

**10.** Use of a hydrogel according to claims 1-6 or a composition according to claim 8 for in vitro cell culture, differentiation or proliferation.

**11.** Use according to claim 10, wherein said cells are adult stem cells isolated from adipose tissue and from human umbilical cord endothelial cells (HUVEC).

**12.** Use of a hydrogel according to claims 1-6 or a composition according to claim 7_for *in vitro* regeneration of a soft tissue.

**13.** Use according to claim 12, wherein said soft tissue is adipose tissue, vascular tissue or skin.

**14.** Carrier of cells containing a hydrogel according to claims 1-6 or a composition according to claim 7.

**15.** Implant or grafting of a cell-containing an hydrogel according to claims 1-6 or a composition according to claim 7.


**Patentansprüche**

**1.** Hydrogel von Gelatine, vernetzt mit Bisacrylamiden primärer oder sekundärer Amine, die ausgewählt sind aus N,N'-Methylen-bisacrylamid (MBA), N,N'-Ethylen-bisacrylamid, Bisacryloylpiperazin, Bisacryloylcystamin und N,N'-Polyoxyalkylen-bisacrylamid, wobei das Hydrogel erhältlich ist durch ein Additionsreaktionsverfahren vom Michael-Typ über die nucleophile Addition freier Aminogruppen in Gelatine und die $\alpha,\beta$-ungesättigte Carbonylgruppe im ausgewählten Bisacrylamid, wobei das Verfahren die folgenden Schritte umfasst:

(a) Umsetzung von Gelatine mit Bisacrylamid in einem Wassermedium;
(b) Reinigung des in Schritt (a) erhaltenen Produkts;
(c) gegebenenfalls Trocknen oder Lyophilisierung.

**2.** Hydrogel gemäß Anspruch 1, wobei die Reaktion in Schritt (a) umfasst:

(a') Herstellen einer wässrigen Lösung von Gelatine bei einer Temperatur von 30 bis 70°C in Gegenwart einer tertiären Aminverbindung oder einer anorganischen Base, wobei die Gelatinekonzentration zwischen 5 und 40% G/V beträgt;
(b') Zugeben von Bisacrylamid zu der in (a') erhaltenen Lösung in einer solchen Menge, dass das molare Verhältnis zwischen den Aminogruppen, die in der Gelatine vorhanden sind (mM ($NH_2$) -Lysin), und den Acrylamidgruppen, die in dem Bisacrylamid vorhanden sind, von 0,5 bis 2,5 reicht;
(c') Erwärmen der in (b') erhaltenen Lösung bei einer Temperatur von zwischen 30 und 70°C für eine Zeitdauer von 4 bis 24 Stunden.

**3.** Hydrogel gemäß Anspruch 1-2, wobei die Reinigung von Schritt (b) aufeinanderfolgende Waschschritte mit Ethanol, gefolgt von 0,1 M HCl und Wasser umfasst.

**4.** Hydrogel gemäß Anspruch 1-3, wobei das Bisacrylamid N,N'-Methylen-bisacrylamid (MBA) ist.

**5.** Hydrogel gemäß Ansprüchen 1-4, wobei das tertiäre Amin Triethylamin in Mengen ist, die von 0,1 bis 0,3 ml pro Gramm Gelatine reichen.

**6.** Hydrogel gemäß einem der vorangehenden Ansprüche, enthaltend eine biologisch aktive Substanz oder Zellen.

**7.** Zusammensetzung, enthaltend ein Hydrogel gemäß Ansprüchen 1-6 in einem wässrigen Medium oder in trockener Form.

8. Hydrogel gemäß Ansprüchen 1-6 oder eine Zusammensetzung gemäß Anspruch 7 zur Verwendung in der kontrollierten Freisetzung von Wirkstoffen, Polypeptiden oder aktiven Biomolekülen.

9. Hydrogel oder Zusammensetzung gemäß Anspruch 8, wobei die aktiven Biomoleküle Wachstumsfaktoren sind.

10. Verwendung eines Hydrogels gemäß Ansprüchen 1-6 oder einer Zusammensetzung gemäß Anspruch 8 für In-vitro-Zellkultur, -Differenzierung oder -Proliferation.

11. Verwendung gemäß Anspruch 10, wobei die Zellen adulte Stammzellen sind, isoliert aus adipösem Gewebe und aus humanen Nabelschnurendothelzellen (HUVEC).

12. Verwendung eines Hydrogels gemäß Ansprüchen 1-6 oder einer Zusammensetzung gemäß Anspruch 7 zur In-vitro-Regenerierung eines Weichgewebes.

13. Verwendung gemäß Anspruch 12, wobei das Weichgewebe adipöses Gewebe, vaskuläres Gewebe oder Haut ist.

14. Zellenträger, enthaltend ein Hydrogel gemäß Ansprüchen 1-6 oder eine Zusammensetzung gemäß Anspruch 7.

15. Implantat oder Transplantat aus einem zellhaltigen Hydrogel gemäß Ansprüchen 1-6 oder einer Zusammensetzung gemäß Anspruch 7.

## Revendications

1. Hydrogel de gélatine réticulée avec des bisacrylamides d'amines primaires ou secondaires qui sont choisis parmi le N,N'-méthylène-bisacrylamide (MBA), le N,N'-éthylène-biscarylamide, la bisacryloylpipérazine, la bisacryloylcystamine et un N,N'-polyoxyalkylène-bisacrylamide, dans lequel ledit hydrogel peut être obtenu par un processus de réaction d'addition de type Michael via l'addition nucléophile de groupes amino libres dans de la gélatine et le groupe carbonyle $\alpha,\beta$ insaturé dans le bisacrylamide choisi, ledit processus comprenant les étapes suivantes :

   (a) réaction de la gélatine avec le bisacrylamide dans un milieu aqueux ;
   (b) purification du produit obtenu dans l'étape (a) ;
   (c) séchage ou lyophilisation optionnel.

2. Hydrogel selon la revendication 1, dans lequel la réaction de l'étape (a) comprend :

   (a') la préparation d'une solution aqueuse de gélatine à une température de 30 à 70 °C en présence d'un composé amine tertiaire ou d'une base inorganique, où la concentration en gélatine se situe entre 5 et 40 % en poids/volume ;
   (b') l'addition d'un bisacrylamide à la solution obtenue en (a') en quantité telle que le rapport molaire entre les groupes amino présents dans la gélatine (mM ($NH_2$) lysine) et les groupes acrylamide présents dans le bisacrylamide se situe dans la plage allant de 0,5 à 2,5 ;
   (c') chauffage de la solution obtenue en (b') à une température entre 30 et 70 °C durant une période de temps de 4 à 24 heures.

3. Hydrogel selon la revendication 1 ou 2, dans lequel la purification de l'étape (b) comprend des lavages consécutifs avec de l'éthanol suivi par HCl 0,1 M et par de l'eau.

4. Hydrogel selon la revendication 1 à 3, dans lequel ledit bisacrylamide est le N,N'-méthylène-bisacrylamide (MBA).

5. Hydrogel selon les revendications 1 à 4 dans lequel ladite amine tertiaire est la triéthylamine en des quantités se situant dans la plage allant de 0,1 à 0,3 ml par gramme de gélatine.

6. Hydrogel selon l'une quelconque des revendications précédentes, contenant une substance biologiquement active ou des cellules.

**7.** Composition contenant un hydrogel selon les revendications 1 à 6 dans un milieu aqueux ou sous forme anhydre.

**8.** Hydrogel selon les revendications 1 à 6 ou composition selon la revendication 7 pour l'utilisation dans la libération contrôlée de médicaments, de polypeptides ou de biomolécules actives.

**9.** Hydrogel ou composition selon la revendication 8, dans lequel lesdites biomolécules actives sont des facteurs de croissance.

**10.** Utilisation d'un hydrogel selon les revendications 1 à 6 ou d'une composition selon la revendication 8 pour la culture, la différenciation ou la prolifération cellulaire in vitro.

**11.** Utilisation selon la revendication 10, dans laquelle lesdites cellules sont des cellules souches adultes isolées de tissu adipeux et de cellules endothéliales de cordon ombilical humain (HUVEC).

**12.** Utilisation d'un hydrogel selon les revendications 1 à 6 ou d'une composition selon la revendication 7 pour la régénération *in vitro* d'un tissu mou.

**13.** Utilisation selon la revendication 12, dans laquelle ledit tissu mou est le tissu adipeux, le tissu vasculaire ou la peau.

**14.** Transporteur de cellules contenant un hydrogel selon les revendications 1 à 6 ou une composition selon la revendication 7.

**15.** Implant ou greffage d'un hydrogel contenant une cellule selon les revendications 1 à 6 ou d'une composition selon la revendication 7.

EP 2 714 109 B1

Figure 1

# Figure 2

15MBA23    15MBA47    25MBA47

Wavenumber [cm$^{-1}$]

## Figure 3

**(MBA-HEX)**

**(GEL 15MBA23)**

## Figure 4

## Figure 5

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010060104 A2
- WO 2008121422 A2

### Non-patent literature cited in the description

- **KUIJPERS A.J. et al.** *Journal of Biomaterials Science, Polymer Edition,* 2000, vol. 11 (3), 225-243
- **A.J. KUIJPERS et al.** *J Biomater Sci Polymer Edn,* 2000, vol. 11 (3), 225-24
- **P. DUBRUEL et al.** *Biomacromolecules,* 2007, vol. 8, 338-344
- **S. VAN VLIERBERGHE et al.** *Biomacromolecules,* 2007, vol. 8, 331-337
- **SADEGHI M et al.** *Materials,* vol. 4 (3), 543-552
- **KUMARI M et al.** *Journal of Applied Polymer Science,* vol. 119 (1), 363-370
- **VAN VLIERBERGHE S et al.** *Biomacromolecules,* 2007, vol. 8 (2), 331-337
- **CAYOT P. et al.** *Analytical Biochemistry,* 1997, vol. 249, 184-200
- **RIGOTTI et al.** Clinical treatment of radiotherapy tissue damage by lipoaspirate transplant: a healing process mediated by adipose derived adult stem cells. *Plast Reconstr Surg.,* 2007, vol. 119 (5), 1409-1422
- **TANZI MC et al.** Adipose tissue engineering: state of the art, recent advances and innovative approaches. *Expert Rev Med Devices,* 2009, vol. 6 (5), 533-51
- **BANDIERA A et al.** *Biotechnol Appl Biochem.,* 2005, vol. 42, 247-56